Europäisches Patentamt

European Patent Office

Office européen des brevets

(11) Veröffentlichungsnummer: **0 309 911**
A1

# (12) EUROPÄISCHE PATENTANMELDUNG

(21) Anmeldenummer: 88115567.5

(51) Int. Cl.⁴: **C07D 307/06**

(22) Anmeldetag: 22.09.88

(30) Priorität: 30.09.87 DE 3732950

(43) Veröffentlichungstag der Anmeldung:
05.04.89 Patentblatt 89/14

(84) Benannte Vertragsstaaten:
BE DE FR GB NL

(71) Anmelder: BASF Aktiengesellschaft
Carl-Bosch-Strasse 38
D-6700 Ludwigshafen(DE)

(72) Erfinder: Hoelderich, Wolfgang, Dr.
Mannheimer Strasse 18 c
D-6710 Frankenthal(DE)
Erfinder: Schwarzmann, Matthias, Dr.
Carl-Bosch-Strasse 54
D-6703 Limburgerhof(DE)

(54) Verfahren zur Herstellung von Tetrahydrofuranen aus 1,4-Butandiolen.

(57) Die Erfindung betrifft ein Verfahren zur Herstellung von Tetrahydrofuranen aus 1,4-Butandiolen, die am C-Gerüst durch Alkyl-, Alkenyl- und/oder Arylreste substituiert sein können, in der Gasphase. Die Umsetzung wird in Gegenwart von Bor- und/oder Eisensilikatzeolith vorzugsweise des Pentasiltyps als Katalysator ausgeführt.

Man kann als Katalysatoren auch mit Alkalimetallen und/oder Erdalkalimetallen und/oder mit Übergangsmetallen und/oder mit Seltenen Erden und/oder Edelmetallen dotierte Zeolithe verwenden.

EP 0 309 911 A1

## Verfahren zur Herstellung von Tetrahydrofuranen aus 1,4-Butandiolen

Die Erfindung betrifft ein Verfahren zur Herstellung von Tetrahydrofuranen aus 1,4-Butandiolen in der Gasphase.

Es ist bekannt, daß man Tetrahydrofuran aus 1,4-Butandiol in der Flüssigphase in Gegenwart von sauren Homogenkatalysatoren wie $H_2SO_4$, $H_3PO_4$, Toluolsulfonsäure mit hohen Ausbeuten von etwa 95 % herstellen kann. Nachteilig ist hierbei bei Abtrennung des Katalysators.

Auch sulfonsäuregruppenhaltige Kationenaustauscher-Harze können für diese intramolekulare Dehydratisierung eingesetzt werden. Hierbei werden 92 bis 95 % Ausbeute erzielt (Houben-Weyl, Bd. Sauerstoffverbindungen I Teil 3, Georg Thieme Verlag, 1965).

Die Umsetzunhg von Diolen an Aluminosilikatzeolithen vom X- und Y-Typ ist ebenfalls bekannt (A. Molnar et al., Proc. ZEOCAT Symp. Siofok, Ungarn, 1985, Acta Physica et Chemica Szegediensis, Sszeged, 1985, S. 571). Neben der gewünschten Dehydratisierung tritt hierbei auch selektivitätsmindernde Fragmentierung des C-Gerüstes auf.

Es bestand daher die technische Aufgabe, Tetrahydrofurane aus 1,4-Butandiolen in sehr hohen Ausbeuten an Katalysatoren zu synthetisieren, die sich durch lange Katalysatorstandzeiten auszeichnen.

Es wurde nun gefunden, daß man Tetrahydrofurane aus 1,4-Butandiolen, die am C-Gerüst durch Alkyl-, Alkenyl- und/oder Arylreste substituiert sein können, in der Gasphase in guten Ausbeuten und bei langen Katalysatorstandzeiten erhält, wenn man die Umsetzung in Gegenwart eines Bor- und/oder Eisensilikatzeolithen als Katalysator ausführt.

Als Ausgangsstoffe kann man 1,4-Butandiole, die in 1, 2, 3 und/oder 4 Position ein oder mehrere lineare, verzweigte oder cyclische Alkyl- und/oder Alkenylreste mit 1 bis 6 Kohlenstoffatomen besitzen, oder 1,4-Butandiole, die ausschließlich oder neben den Alkyl- bzw. Alkenylresten Aryl- und/oder Alkylaryl- und/oder Aralkylreste mit 6 bis 12 Kohlenstoffatomen besitzen, und 1,4-Butandiol verwenden.

Als Katalysatoren für das erfindungsgemäße Verfahren werden Bor- und Eisensilikatzeolithe eingesetzt, insbesondere kommen in Betracht Bor- und Eisensilikatzeolithe vom Pentasiltyp. Diese haben als Grundbaustein eine aus $SiO_4$-Tetraedern aufgebauten Fünfring gemeinsam.

Die Borosilikatzeolithe werden z.B. bei 90 bis 200 °C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentramin-Lösung mit und insbesondere ohne Alkali- oder Erdalkalizusatz zur Reaktion bringt. Auch die isotaktischen Zeolithe nach DE-OS 3 006 471 sind geeignet. Die Borosilikatzeolithe können statt in wäßriger Aminlösung auch in etherischer Lösung, z.B. Diethylenglykoldimethylether oder in alkoholischer Lösung, z.B. 1,6-Hexandiol hergestellt werden.

Die Eisensilikatzeolithe erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Die so hergestellten Boro- und Eisensilikatzeolithe können nach ihrer Isolierung, Trocknung bei 100 bis 160 °C, vorzugsweise 110 °C und Calcinierung bei 450 und 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminiumsilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn die isolierten Boro- bzw. Eisensilikatzeolithe direkt nach der Trocknung verformt werden und erst nach der Verformung einer Calcinierung unterworfen werden. Die Boro- und Eisensilikzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Liegen die Zeolithe aufgrund der Art der Herstellung nicht in der aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann man die Zeolithe auch modifizieren, z.B. indem man die unverformten oder verformten Zeolithe mit Metallsalzen durch einen Ionenaustausch oder durch Imprägnierung dotiert. Als Metalle kommen Alkalimetalle wie Li, Cs, Erdalkalimetalle wie Mg, Ca, Übergangsmetalle wie Cu, Fe, Zn, Co und Edelmetalle wie Pd, Pt, Seltenen Erdmetalle wie Ce, La in Frage.

Zweckmäßigerweise führt man die Dotierung so durch, daß man die verformten Zeolithe in einem Steigrohr vorlegt und bei 20 bis 100°C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf die Zeolithe ist gegeben, indem man das zeolithische Material mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃) x 6 H₂O oder Wolframsäure oder Cs₂CO₃ in Wasser löst. Mit dieser Lösung werden die verformten oder unverformten Zeolithe eine gewisse Zeit, etwa 30 Minuten, getränkt. Die überstehende Lösung kann am Rotationsverdampfer von Wasser befreit werden. Danach wird der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige Ni(NO₃)₂-Lösung oder ammoniakalische Pd(NO₃)₂-Lösung oder Ammoniumvanadat herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch der in der H-Form, Ammonium-Form oder Alkali-Form vorliegenden Zeolithe kann so vorgenommen werden, daß man die Zeolithe in Strängen oder Pellets in einer Kolonne vorlegt und darüber z.B. eine wäßrige Ni(CO₃)₂-Lösung oder ammoniakalische Pd(NO₃)₂-Lösung bei leicht erhöhter Temperatur zwischen 30 und 80°C 15 bis 20 h im Kreislauf leitet. Danach wird mit Wasser ausgewaschen, bei etwa 150°C getrocknet und bei etwa 550°C calciniert. Bei manchen metalldotierten Zeolithen z.B. Pd-, Cu-, Ni-dotierten Zeolithen ist eine Nachbehandlung mit Wasser vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft so vor, daß man Zeolithe in Pulverform im 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 Gew.%igen, insbesondere 10 bis 20 Gew.%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von 0,5 bis 5, vorzugsweise 1 bis 3 h. Nach Isolierung, z.B. durch Abfiltrieren und Auswaschen, wird das zeolithische Material zweckmäßig bei Temperaturen von 100 bis 160°C getrocknet und bei Temperaturen von 450 bis 600°C calciniert. Gemäß einer weiteren bevorzugten Ausführungsform für die Säurebehandlung wird das zeolithische Material nach einer Verformung mit Bindemittel bei erhöhter Temperatur, bei Temperaturen von 50°C bis 90°C, insbesondere 60°C bis 80°C, über einen Zeitraum von 0,5 bis 5, insbesondere mit 12 bis 20 Gew.%iger Salzsäure, behandelt. Anschließend wird das zeolithische Material ausgewaschen und zweckmäßig, bei Temperaturen von 100 bis 160°C, getrocknet und bei Temperaturen von 450 bis 600°C calciniert.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Als vorteilhaft hat sich die Behandlung mit primärem Natriumphosphat erwiesen. Hierbei werden die Zeolithe in Strang-, Tabletten- oder Wirbelgut-Form in wäßriger NaH₂PO₄-Lösung getränkt, bei 110°C getrocknet und bei 500°C calciniert.

Die hier beschriebenen Katalysatoren können wahlweise als 2- bis 4-mm-Stränge oder als Tabletten mit

3

3 bis 5 mm Durchmesser oder als Splitt mit Teilchengrößen von 0,1 bis 0,5 mm oder als Wirbelkontakt eingesetzt werden.

Die Umsetzung wird vorzugsweise in der Gasphase bei 100 bis 500°C, insbesondere bei 150 bis 300°C, und einer Belastung WHSV = 0,1 bis 20 h⁻¹, insbesondere 0,5 bis 5 h⁻¹ (g Einsatzgemisch/g Katalysator und Stunde) ausgeführt. Die Reaktion kann in einem Festbett oder auf- und abbewegenden Bett oder Wirbelbett ausgeführt werden.

Es ist auch möglich, die Reaktion in der Flüssigphase (Suspensions-, Riesel- oder Sumpffahrweise) bei Temperaturen zwischen 50 und 200°C durchzuführen.

Das Verfahren kann bei Normaldruck, bei vermindertem oder erhöhtem Druck, vorzugsweise kontinuierlich durchgeführt werden.

Schwerflüchtige oder feste Ausgangsstoffe werden in gelöster Form, z.B. in THF-, Toluol- oder Petrolether-Lösung umgesetzt. Allgemein ist eine Verdünnung des Ausgangsstoffes mit derartigen Lösungsmitteln oder mit Inertgasen wie N₂, Ar, H₂O-Dampf möglich.

Nach der Umsetzung werden die entstandenen Produkte durch übliche Verfahren z.B. durch Destillation aus dem Reaktionsgemisch isoliert; nichtumgesetztes Einsatzgemisch wird gegebenenfalls, in die Umsetzung zurückgeführt.

Beispiele 1 - 6

Die Reaktionen in der Gasphase werden unter isothermen Bedingungen in einem Rohrreaktor (Wendel, 0,6 cm Innendurchmesser, 90 cm Länge) im Verlauf von mindestens 6 Stunden durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem SiO₂, 122 g H₃BO₃, 8000 g einer wäßrigen 1,6-Hexandiamin-Lösung (Mischung 50:50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% SiO₂ und 2,3 Gew.% B₂O₃.

Mit diesem Material werden durch Verformen mit einem Verformungshilfsmittel 2-mml-Stränge hergestellt, die bei 110°C/16 h getrocknet und bei 500°C/24 h calciniert werden.

Katalysator B

Der Eisensilikatzeolith des Pentasil-Typs wird unter hydrothermalen Bedingungen bei autogenem Druck und 165°C aus 273 g Wasserglas, gelöst in 253 g einer wäßrigen 1,6 Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110°C/24 h getrocknet und bei 500°C/24 h calciniert. Man erhält Eisensilikatzeolith mit einem SiO₂/Fe₂O₃-Verhältnis von 17,7 und einen Na₂O-Gehalt von 1,2 Gew.%. Der Katalysator wird zu 2,5-mm-Strängen verstrangt, bei 110°C/16 h getrocknet und bei 500°C/24 h calciniert.

Katalysator C

Der Borosilikatzeolith (siehe Katalysator A) wird mit Boehmit im Gewichtsverhältnis 60:40 zu 2 mm Strängen verformt, die bei 110°C/16 h getrocknet und bei 500°C/24 h calciniert werden.

Diese Stränge werden mit wäßriger H₂WO₄-Lösung imprägniert, bei 130°C/2 h getrocknet und bei 540°C/2 h calciniert. Der W-Gehalt beträgt 3,1 Gew.%.

Katalysator D

Katalysator A wird mit wäßriger Ce(NO₃)₂-Lösung imprägniert, bei 130°C/2 h getrocknet und bei 540°C/2 h calciniert. Der Ce-Gehalt beträgt 1,8 Gew.%. Die mit diesen Katalysatoren erhaltenen Versuchsergebnisse und Reaktionsbedingungen sind in der nachfolgenden Tabelle beschrieben.

Tabelle

| 1,4-Butandiol ----→ Tetrahydrofuran + $H_2O$ | | | | | | |
|---|---|---|---|---|---|---|
| Beispiel | 1 | 1 | 3 | 4 | 5 | 6 |
| Katalysator | A | A | B | C | C | D |
| Temperatur °C | 200 | 300 | 200 | 200 | 300 | 200 |
| WHSV h⁻¹ | 1 | 2 | 1 | 2 | 2 | 1 |
| Umsatz % | 100 | 100 | 100 | 100 | 100 | 100 |
| Selektivität % | 99,5 | 98,3 | 97,6 | 99,8 | 98,6 | 98,7 |

## Ansprüche

1. Verfahren zur Herstellung von Tetrahydrofuranen aus 1,4-Butandiolen, die am C-Gerüst durch Alkyl-, Alkenyl- und/oder Arylreste substituiert sein können, in der Gasphase, dadurch gekennzeichnet, daß man die Umsetzung in Gegenwart von Bor- und/oder Eisensilikatzeolith als Katalysator ausführt.

2. Verfahren nach Anspruch 1, dadurch gekennzeichnet, daß man Zeolithe des Pentasiltyps verwendet.

3. Verfahren nach Anspruch 1, dadurch gekennzeichnet, daß man als Katalysator mit Alkalimetallen und/oder Erdalkalimetallen und/oder mit Übergangsmetallen und/oder mit Seltenen Erden und/oder Edelmetallen dotierte Zeolithe verwendet.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 461 922 (GENERAL ELECTRIC CO.) * Insgesamt * --- | 1-3 | C 07 D 307/06 |
| Y | CHEMICAL ABSTRACTS, Band 84, Nr. 13, 29. März 1976, Seite 511, Zusammenfassung Nr. 89981a, Columbus, Ohio, US; & SU-A-296 414 (KH.I. ARESHIDZE et al.) 05-12-1975 * Zusammenfassung * --- | 1-3 | |
| Y | EP-A-0 232 712 (BASF) * Insgesamt; insbesondere Seite 3, Zeilen 28-57; Seite 4, Zeilen 1-3 * ----- | 1-3 | |
| | | | **RECHERCHIERTE SACHGEBIETE (Int. Cl.4)** |
| | | | C 07 D 307/00 |

Der vorliegende Recherchenbericht wurde für alle Patentansprüche erstellt

| Recherchenort | Abschlußdatum der Recherche | Prüfer |
|---|---|---|
| DEN HAAG | 16-12-1988 | ALLARD M.S. |